# EUROPÄISCHE PATENTSCHRIFT

(11) **EP 3 033 019 B1**
(45) Veröffentlichungstag und Bekanntmachung des Hinweises auf die Patenterteilung: **18.08.2021**
(21) Anmeldenummer: 15715261.2
(22) Anmeldetag: 09.04.2015
(51) Int. Cl.: A61B 17/80, A61B 17/56, A61B 17/00

(54) **PRAEFORMIERTES IMPLANTAT ZUR REPOSITION UND FIXATION EINER ZENTROLATERALEN MITTELGESICHTSFRAKTUR**
PRE-FORMED IMPLANT FOR REPOSITIONING AND FIXATION OF A CENTROLATERAL MIDFACIAL FRACTURE
IMPLANT PRÉFORMÉ POUR LE REPOSITIONNEMENT ET LA FIXATION D'UNE FRACTURE DU VISAGE MÉDIAN CENTROLATÉRAL

(30) Priorität: 11.04.2014 DE 102014005346; 11.04.2014 DE 202014103858 U
(43) Veröffentlichungstag der Anmeldung: 22.06.2016
(73) Patentinhaber: Karl Leibinger Medizintechnik GmbH & Co. KG, 78570 Mühlheim/Donau (DE)
(72) Erfinder: LEIBINGER, Christian, 78570 Mühlheim (DE); MARTIN, Michael, 78570 Mühlheim (DE)
(74) Vertreter: Winter, Brandl - Partnerschaft mbB
(86) Internationale Anmeldenummer: PCT/EP2015/057738
(87) Internationale Veröffentlichungsnummer: WO 2015/155296

(56) Entgegenhaltungen:
- EP-A1- 1 175 181
- EP-A1- 2 201 899
- EP-A1- 2 698 122
- WO-A1-2012/079611

## Beschreibung

Die Erfindung betrifft ein Implantat zur Reposition und Fixation einer zentrolateralen Mittelgesichtsfraktur.

Zentrolaterale Mittelgesichtsfrakturen, wie beispielsweise Jochbeinfrakturen und Le Fort-Frakturen, insbesondere Le Fort I-Frakturen und Le Fort II-Frakturen, werden meist durch eine direkte Gewalteinwirkung hervorgerufen, wobei es sich dabei regelmäßig um eine Komplexfraktur handelt, bei welcher die Bruchenden gegeneinander verschoben werden, so dass eine Dislokation der Fragmente auftritt.

Bei der Therapie einer solchen dislozierten Fraktur ist in der Regel ein chirurgischer Eingriff notwendig, wobei mit Hilfe eines Implantats eine Fixation und Reposition der Knochenfragmente hergestellt wird. Als Implantate werden dabei üblicherweise plane, plattenförmige Osteosyntheseplatten aus Titan oder anderen geeigneten Materialien verwendet, welche in verschiedenen Formen und Stärken erhältlich sind. Diese Titanplatten werden zumeist mit Schrauben an Knochenfragmenten angebracht und bieten daher eine Stütze, damit die Fragmente wieder in ihrer ursprünglichen Lage gehalten werden, um so den Heilungsprozess des Jochbeins zu ermöglichen. Während eines solchen operativen Eingriffs (Osteosynthese) ist es notwendig, dass die plan ausgebildeten Implantate vom behandelten Chirurgen manuell an die anatomischen, knochengeometrischen Strukturen des Implantatempfängers mittels Biegen angepasst werden. Diese individuelle Anpassung des Implantats ist zeitaufwendig und kann dazu führen, dass das Implantat mehrmals angepasst werden muss, bis es schließlich die gewünschte Form angenommen hat bzw. an den anatomischen, knochengeometrischen Strukturen des Implantatempfängers derart anliegt, dass diese adäquat reponiert und fixiert sind und sich daher nicht mehr gegeneinander verschieben können. Ferner kann es aufgrund der Anpassung des Implantats durch Biegen zu einer Vorschwächung, insbesondere an den Biegezonen, kommen.

Ein weiterer Nachteil vorbekannter planer Osteosyntheseplatten besteht darin, dass diese nur eingeschränkt für die Reposition und Fixation von Trümmerbrüchen (Mehrfragmentfrakturen), insbesondere von zentrolateralen Mittelgesichtsfrakturen Typ V, geeignet sind. Während eines gesichtschirurgischen Eingriffs zur Behandlung eines solchen Trümmerbruchs ist es häufig nicht oder kaum möglich, mittels vorbekannter Implantate alle oder zumindest den Großteil der einzelnen Knochenfragmente derart zu reponieren und/oder zu fixieren, dass die Normpositionen der einzelnen Fragmente vollständig oder zumindest annähernd vollständig wiederherstellbar ist. Daher sind vorbekannte Osteosyntheseplatten grundsätzlich nicht zur Überbrückung von mehrfachen, komplexen Frakturen geeignet

Gattungsgemäßer Stand der Technik ist aus der europäischen Patentanmeldung EP 2 698 122 A1 bekannt. Diese offenbart ein orthognatisches Implantat mit einer Brücke und verschiedenen fingerförmigen Segmenten.

Zudem wird in der WO 2012/079 611 A1 eine chirurgische Vorrichtung zum Herstellen eines Implantats durch Biegen offenbart, wobei eine Form der Vorrichtung eine patientenspezifische Form besitzt, die basierend auf anatomischen Daten des bestimmten Patienten individuell ausgebildet ist.

Weiterhin sind auch die Dokumente WO 00/66012 A und EP 2 201 899 A1 als Stand der Technik anzusehen.

Es besteht daher die Aufgabe, ein Implantat zur verbesserten anatomischen Reposition und Osteosynthese einer zentrolateralen Mittelgesichtsfraktur bereitzustellen, welches keine oder kaum eine manuelle Anpassung an die anatomischen, knochengeometrischen Strukturen eines Implantatempfängers notwendig macht.

Die der Erfindung zugrunde liegende Aufgabe wird durch die Merkmale aus Anspruch 1 und zwar dadurch gelöst, dass das Implantat mehrere präformierte Segmente, vorzugsweise drei oder mehr präformierte Segmente umfasst, wobei die Form eines ersten Segments derart ausgebildet ist, dass seine am Knochen anzuliegende Unterseite mit der anatomischen Struktur eines Teilbereiches der Außenseite eines Oberkieferknochens (Maxilla) komplementär ist, wobei die Form eines zweiten Segments derart ausgebildet ist, dass seine am Knochen anzuliegende Unterseite mit der außenseitigen anatomischen Struktur eines Stegbereichs zwischen Oberkiefer und Jochbein (Crista zygomaticoalveolaris) komplementär ist, und wobei die Form eines dritten Segments derart ausgebildet ist, dass eine am Knochen anzuliegende Unterseite mit der anatomischen Struktur zumindest eines außenseitigen Teilbereiches eines Jochbeins (Os zygomaticum, Os jugale) komplementär ist.

Außenseite bzw. außenseitig bezieht sich dabei auf die von außen sichtbare Seite des Schädels relativ zum Schädelmittelpunkt. In reponierter Stellung bzw. Normposition der Knochenfragmente ist vorgesehen, dass alle Segmente, des erfindungsgemäßen Implantats komplementär zur Außenseite des Knochens anliegen.

Das erfindungsgemäße Implantat hat insbesondere den Vorteil gegenüber vorbekannten Implantaten zur Reposition und Fixation einer zentrolateralen Mittelgesichtsfraktur, dass es bereits präformiert vorliegt und an die anatomischen Strukturen der zentrolateralen Mittelgesichtsknochen des Implantatempfängers angepasst ist. Normalerweise müssen vorbekannte Implantate, wie bereits erwähnt, während eines gesichtschirurgischen Eingriffs durch den Chirurgen mittels Biegen an die anatomischen Strukturen des Implantatempfängers angepasst werden. Dieser Vorgang ist zeitaufwendig und kann das Material des Implantats, insbesondere an den Biegestellen, schwächen.

Häufig besteht bei einer zentrolateralen Mittelgesichtsfraktur eine Mehrfragmentfraktur. Durch die Vorgabe des erfindungsgemäßen präformierten Implantats lassen sich einzelne Fragmente regelrecht reponieren und fixieren, so dass die ursprüngliche, dreidimensionale Ausrichtung der zentrolateralen Mittelgesichtsknochen, insbesondere des Jochbeins, gewährleistet ist.

Anhand von Untersuchungen der Schädelform hat sich gezeigt, dass die individuelle Formabweichung im Bereich der zentrolateralen Mittelgesichtsknochen, insbesondere des Jochbeins, nur minimal ist. Daher ist es möglich, ein präformiertes Implantat auszubilden, welches vollständig oder teilweise komplementär zu diesen bei allen Menschen (einer ethnischen Gruppe) ähnlichen anatomischen Strukturen ist. Lediglich die Schädelgröße sowie die ethnische Herkunft des Implantatempfängers müssen bei der Wahl einer bestimmten Implantatgröße berücksichtigt werden. Eine vorgeformte dreidimensionale Ausgestaltung ermöglicht dabei auch bei beidseitigen Gesichtsfrakturen eine adäquate Reposition und Fixation der Knochenfragmente.

Dabei ist es besonders zweckmäßig, dass das erste Segment derart ausgeformt ist, dass es komplementär zu zumindest einem Ankerpunkt, vorzugsweise zu zumindest zwei Ankerpunkten, der Außenseite des Oberkieferknochens ausgeformt ist und das gesamte erfindungsgemäße Implantat, insbesondere die einzelnen Segmente des erfindungsgemäßen Implantats, anhand des ersten Segmentes in eine für die nachfolgende Reposition von Knochenfragmenten geeignete Lage ausrichtbar ist/sind. Das bedeutet, dass eine korrekte Ausrichtung zumindest des ersten Segmentes mittels zumindest eines Ankerpunktes ausreichend ist, um das gesamte erfindungsgemäße Implantat korrekt auszurichten, wodurch Knochenfragmente in ihrer Normposition reponiert und fixiert werden können. Die Form des ersten Segments kann beispielsweise derart ausgeformt sein, dass wenigstens seine am Knochen anliegende Unterseite mit der anatomischen Struktur des Bereichs, welcher oberhalb des Alveolarfortsatzes des Oberkiefers angrenzt, insbesondere des Zahnwurzelbereichs (Limbus alveolaris), vollständig oder teilweise komplementär ist.

Es kann vorgesehen sein, dass die Form des zweiten Segments derart ausgeformt ist, dass wenigstens seine am Knochen anliegende Unterseite mit der außenseitigen anatomischen Struktur des lateralen Jochbeinpfeilers vollständig oder teilweise komplementär ist.

Es kann vorgesehen sein, dass die Form des dritten Segments derart ausgeformt ist, dass wenigstens seine am Knochen anliegende Unterseite mit der außenseitigen anatomischen Struktur der Jochbeinprominenz (Processus zygomaticus) vollständig oder teilweise komplementär ist.

Das erfindungsgemäße Implantat kann ferner ein viertes und/oder ein fünftes Segment umfassen, wobei die Form des vierten Segments derart ausgebildet ist, dass wenigstens seine am Knochen anliegende Unterseite mit der anatomischen Struktur zumindest eines Teilbereiches der Außenseite eines Oberkieferknochens, insbesondere mit der anatomischen Struktur zumindest eines Teilbereiches der Spina nasalis, vollständig oder teilweise komplementär ist, und/oder wobei die Form des fünften Segments derart ausgebildet ist, dass wenigstens seine am Knochen anliegende Unterseite mit der außenseitigen anatomischen Struktur zumindest eines Teilbereiches eines paranasalen Stegbereichs zwischen Oberkiefer und Jochbein vollständig oder teilweise komplementär ist.

Durch die Erweiterung des erfindungsgemäßen Implantats um ein viertes und/oder ein fünftes Segment ist es möglich, komplexere Frakturen (insbesondere komplexe Mehrfragmentfrakturen), beispielsweise Jochbeinfrakturen oder Le Fort-Frakturen, insbesondere Le Fort I-Frakturen und Le Fort II-Frakturen, zu reponieren und zu fixieren. Die Ausrichtung des gesamten Implantats wird dabei vorzugsweise anhand des ersten Segments und/oder der Verlängerung des ersten Segments, welche hierin als viertes Segment bezeichnet ist, bestimmt. Es ist insbesondere zweckmäßig, dass die Spina nasalis als Ankerpunkt des optionalen vierten Segments dient, dessen Unterseite zur Spina nasalis vollständig oder teilweise komplementär ist. Durch das optionale fünfte Segment des erfindungsgemäßen Implantats können beispielsweise Knochenfragmente reponiert und fixiert werden, die bei Le Fort-Frakturen, insbesondere Le Fort I-Frakturen, vorkommen.

Die mehrsegmentige Anordnung des erfindungsgemäßen Implantats erlaubt eine sichere Insertion des präformierten Implantats während einer Operation, da sich das Implantat mittels des ersten und/oder des vierten Segments präzise im Bereich des Oberkiefers platzieren lässt und dadurch die Platzierung der weiteren Segmente, vorzugsweise des zweiten und des dritten Segmentes, insbesondere des zweiten, dritten und fünften Segments, festgelegt wird. Durch die einzelnen Segmente werden somit die ursprünglichen Positionen der Knochenfragmente vorgegeben.

Es ist vorstellbar, dass das erfindungsgemäße Implantat mit allen fünf Segmenten vorgefertigt vorliegt und dass die einzelnen Segmente, zum Beispiel durch den Chirurgen, je nach Bedarf, beispielsweise mittels einer Drahtschere, entfernbar sind, so dass das erfindungsgemäße Implantat jeweils ausschließlich diejenigen präformierten Segmente umfasst, welche zur Reposition und Fixation der jeweiligen zentrolateralen Mittelgesichtsfraktur notwendig sind.

Um Knochenfragmente, insbesondere bei Mehrfragmentfrakturen, reponieren und fixieren zu können, ist vorgesehen, dass das erfindungsgemäße Implantat plattenförmig und zumindest teilweise gekrümmt ausgebildet ist, so dass das Implantat eine dreidimensionale Ausbildung aufweist. Die Krümmung des Implantats ist dabei an die anatomischen Strukturen zumindest von Teilbereichen der zentrolateralen Mittelgesichtsknochen angepasst. So können zumindest Teilbereiche der gesunden (in Normposition vorliegenden) zentrolateralen Mittelgesichtsknochen als Matrize bei der Herstellung des erfindungsgemäßen Implantats verwendet werden.

Zur Determination der jeweiligen Größe des erfindungsgemäßen Implantats bzw. der Implantatsegmente können bestimmte Körperparameter, wie beispielsweise die Schädelgröße des Implantatempfängers, berücksichtigt werden. Kinder und Jugendliche brauchen üblicherweise ein kleineres Implantat als Erwachsene. Dabei ist vorgesehen, dass zur Auswahl der Größe eines einzusetzenden Implantats eine präoperative Auswahl getroffen wird. Es ist ferner vorgesehen, dass mehrere, vorzugsweise zumindest drei, verschiedene Größen eines präformierten Implantats (pro ethnische Gruppe) bereitgestellt sind, so dass anhand dieser Implantate jegliche anatomische Struktur der zentrolateralen Mittelgesichtsknochen aller Patienten in ihrer Normposition wiederherstellbar sind. Geeignete Maßnahmen, um präoperativ die adäquate Größe eines erfindungsgemäßen Implantats zu ermitteln, sind beispielsweise die Verwendung von Computerprogrammen wie zur Verarbeitung von 3D-Datensätzen (zum Beispiel Medtronic, IVS Solutions (Chemnitz), Brainlab (München)), welche durch Computertomografie ermittelt wurden, Vermessung des Augenabstandes, Vermessung des Schädelumfangs oder vorzugsweise Anlegen verschiedener Größen spiegelbildlich ausgeformter Implantate an der intakten Gesichtshälfte des Patienten. Insbesondere mittels des Anlegens verschiedener Größen eines spiegelbildlich ausgeformten Implantats an der intakten Gesichtshälfte kann schnell und einfach ermittelt werden, welche Größe zur Reposition und Fixation einer zentrolateralen Mittelgesichtsfraktur benötigt wird.

Ferner ist/sind die ethnische Herkunft und/oder das Geschlecht des Implantatempfängers entscheidend bei der Ausformung des Implantats. Das bedeutet, dass für unterschiedliche ethnische Bevölkerungsgruppen und/oder Geschlechter auch unterschiedliche Implantate hergestellt bzw. zur Reposition und Fixation einer zentrolateralen Mittelgesichtsfraktur verwendet werden müssen.

Das erfindungsgemäße Implantat hat beispielsweise eine Dicke von 0,2 mm bis 1,0 mm, vorzugsweise von 0,3 mm bis 0,5 mm. Es hat den Vorteil, dass es dünner ausgestaltet sein kann als vorbekannte Implantate, da es nicht oder kaum durch Verbiegen angepasst werden muss. Aufgrund dessen, dass das Implantat bereits präformiert ist, kann es eine erhöhte Steifigkeit gegenüber anderen Implantaten haben.

Es kann vorgesehen sein, dass das erfindungsgemäße Implantat beispielsweise durch Biegen, vorzugsweise lediglich minimal, angepasst werden kann. Dabei kann es vorteilhaft sein, dass das präformierte erfindungsgemäße Implantat weiter Biegestellen umfasst, anhand derer eine zumindest annähernd exakte Anpassung an die anatomischen Strukturen des Implantatempfängers möglich ist. Das Implantat kann zudem auch aus mehreren verschiedenen Materialien mit unterschiedlicher Steifigkeit und Biegsamkeit hergestellt sein.

Um Irritationen und Verletzungen der Weichteile zu verhindern oder zumindest zu vermindern, weist das erfindungsgemäße Implantat eine abgerundete Berandung auf.

Damit durch das erfindungsgemäße Implantat eine adäquate Fixation und Reposition einer zentrolateralen Mittelgesichtsfraktur gewährleistet ist, ist vorgesehen, dass das Implantat ein Fixierungselement oder insbesondere mehrere Fixierungselemente umfasst. Ein Fixierungselement kann zumindest einen Fixierungsabschnitt, wie beispielsweise ein Loch oder mehrere Löcher, insbesondere ein Bohrloch oder mehrere Bohrlöcher, mit oder ohne Innengewinde umfassen. Es kann weiter vorgesehen sein, dass das Fixierungselement zumindest eine Schraube oder mehrere Schrauben, vorzugsweise ausgestattet mit einem in das Innengewinde des zumindest einen Loches passenden Außengewindes oder Außengewindeabschnitts, umfasst. Das eine Fixierungselement oder die Fixierungselemente sind zur Fixierung und Reposition zumindest eines Knochenfragments, vorzugsweise mehrerer Knochenfragmente, ausgebildet. Es ist besonders zweckmäßig, wenn das erfindungsgemäße Implantat mit einem ThreadLock TS-System verwendbar ist bzw. dieses umfasst. Das ThreadLock TS-System ist im Stand der Technik ausführlich beschrieben. Die Schrauben des ThreadLock TS-Systems umfassen zumindest zwei unterschiedliche Außengewinde, wobei beispielsweise das zur Verriegelung dienende Außengewinde am Schraubenkopf korrespondierend zum Innengewinde des zumindest einen Loches des Implantats ist. Mittels des zweiten Außengewindes (Knochengewindes) der Schraube wird diese in den Knochen bzw. das Knochenfragment eingeschraubt und somit im Knochen bzw. Knochenfragment fixiert.

Das erfindungsgemäße Implantat kann aus Titan und/oder einer Titanlegierung und/oder einem oder mehreren Kunststoffen, insbesondere einem oder mehreren resorbierfähigen Materialien, hergestellt sein.

Es kann ferner vorgesehen sein, dass die Vorderseite des erfindungsgemäßen Implantats derart ausgebildet ist, dass sie an die anatomischen Strukturen des Implantatempfängers angepasst ist. Das heißt, dass die Vorder- und Unterseite des erfindungsgemäßen Implantats zumindest annähernd komplementär ausgebildet sind.

Die Erfindung wird nun anhand zweier Ausführungsbeispiele näher beschrieben, ist aber nicht auf diese Ausführungsbeispiele beschränkt. Weitere Ausführungsbeispiele ergeben sich durch Kombination der Merkmale einzelner oder mehrerer Schutzansprüche untereinander und/oder mit einzelnen oder mehreren Merkmalen des Ausführungsbeispiels.

Es zeigt, zum Teil schematisiert:
- Fig. 1: eine Ausführungsform des erfindungsgemäßen Implantats mit drei Segmenten in ihrer Position am menschlichen Schädel,
- Fig. 2: eine Ausführungsform des erfindungsgemäßen Implantats mit fünf Segmenten in ihrer Position am menschlichen Schädel,
- Fig. 3: eine nicht abgeschlossene Auswahl von häufig auftretenden Frakturlinien der zentrolateralen Mittelgesichtsknochen,
- Fig. 4: eine perspektivische Darstellung der Ausführungsform des erfindungsgemäßen Implantats aus Fig. 1,
- Fig. 5: eine perspektivische Darstellung der Ausführungsform des erfindungsgemäßen Implantats aus Fig. 1 und 4.

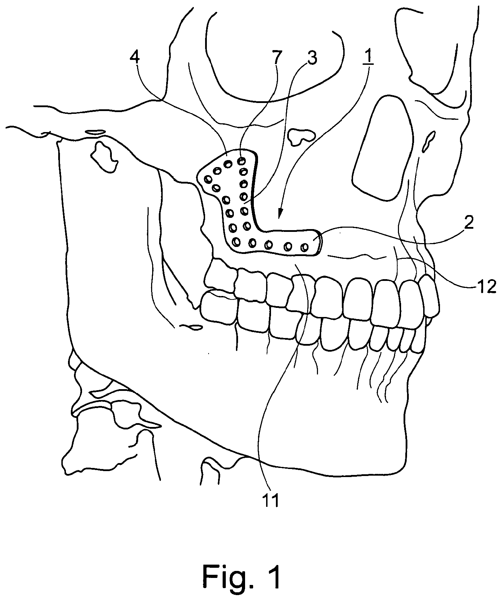

Eine im Ganzen als 1 bezeichnete Ausführungsform des erfindungsgemäßen Implantats ist in Figuren 1, 4 und 5 gezeigt.

In Figur 1 ist zu sehen, wo das präformierte, dreidimensional ausgeformte, plattenförmige Implantat 1 bestehend aus drei Segmenten 2, 3 und 4 in reponierter Stellung der Knochenfragmente außenseitig am Schädel des Implantatempfängers angelegt ist.

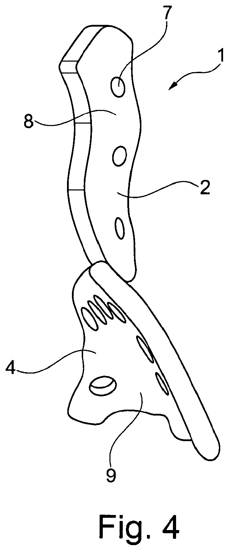

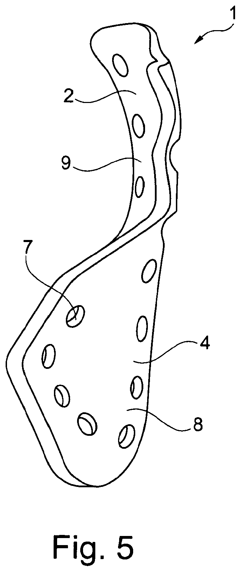

Ein entsprechendes isoliertes Implantat ist in Figur 4 und Figur 5 schematisiert in perspektivischer Ansicht dargestellt.

Dabei ist die Form des ersten Segments 2, welche derart ausgebildet ist, dass wenigstens die am Knochen anliegende Unterseite 9 des Implantats mit der anatomischen Struktur zumindest eines außenseitigen Teilbereiches eines Oberkieferknochens, insbesondere des Zahnwurzelbereiches (Limbus alveolaris) 11, vollständig oder teilweise komplementär ist. Die Form des zweiten Segments 3 ist derart ausgebildet, dass wenigstens seine am Knochen anliegende Unterseite 9 mit der außenseitigen anatomischen Struktur eines Stegbereichs zwischen Oberkiefer und Jochbein (Crista zygomaticoalveolaris) 15, insbesondere des lateralen Jochbeinpfeilers, vollständig oder teilweise komplementär ist. Ferner ist die Form des dritten Segments 4 derart ausgebildet, dass wenigstens seine am Knochen anliegende Unterseite 9 mit der außenseitigen anatomischen Struktur zumindest eines Teilbereiches eines Jochbeins, insbesondere der Jochbeinprominenz 13, vollständig oder teilweise komplementär ist. Das in Figur 1 abgebildete Ausführungsbeispiel ist insbesondere zur Reposition und Fixation einer Jochbeinfraktur geeignet.

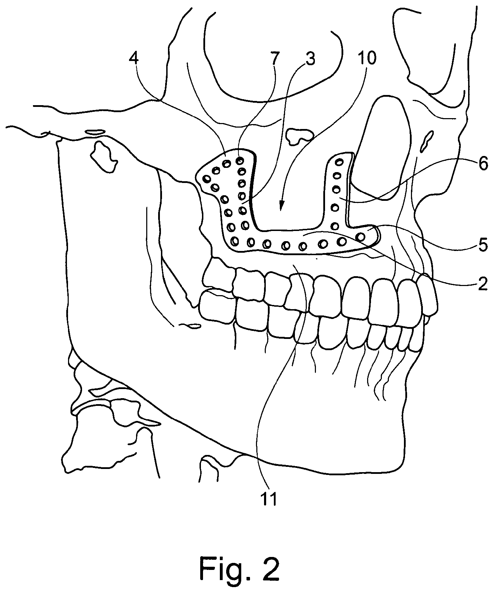

Das in Figur 2 dargestellte präformierte Implantat 10 unterscheidet sich von der in Figur 1 abgebildeten Ausführungsform insofern, dass es weiter ein viertes und ein fünftes Segment 5, 6 umfasst. Die Form des vierten Segments 5 ist derart ausgebildet, dass wenigstens seine am Knochen anliegende Unterseite 9 mit der außenseitigen anatomischen Struktur zumindest eines Teilbereiches eines Oberkiefers, insbesondere mit der anatomischen Struktur zumindest eines Teilbereiches der Spina nasalis 12, vollständig oder teilweise komplementär ist. Die Form des fünften Segments 6 ist ferner derart ausgebildet, dass wenigstens seine außenseitig am Knochen anliegende Unterseite 9 mit der anatomischen Struktur zumindest eines Teilbereiches eines paranasalen Stegbereichs zwischen Oberkiefer und Jochbein vollständig oder teilweise komplementär ist.

Die Figur 2 dargestellte Ausführungsform der Erfindung hat insbesondere den Vorteil, dass damit komplexere Frakturen, wie zum Beispiel Jochbeinfrakturen und Le Fort-Frakturen, insbesondere Le Fort I-Frakturen 17 und Le Fort II-Frakturen 16, gerichtet werden können. Durch das vierte Segment 5 umfasst das Implantat 10 einen zusätzlichen Ankerpunkt, mittels dessen das Implantat 10, insbesondere an der Spina nasalis 12 (siehe Figur 1), ausgerichtet werden kann. Das fünfte Segment 6 ist als paranasale Verlängerung des Implantats 10 ausgeformt, wodurch beispielsweise großflächigere Frakturen leichter und besser reponiert werden können.

Das erfindungsgemäße Implantat 1, 10 ist plattenförmig (mit einer Dicke von 0,3 mm bis 0,5 mm) und zumindest teilweise gekrümmt ausgebildet. Anhand der Krümmungen ist die Form der Segmente des Implantats 1, 10, insbesondere der Unterseite 9 des Implantats 1, 10, an die anatomische Struktur der zentrolateralen Mittelgesichtsknochen (insbesondere des Jochbeins) angepasst, so dass die Segmente zumindest annähernd komplementär außenseitig am jeweiligen Knochen bzw. Knochenbereich anliegen. Um das Implantat 1, 10 regelrecht am Schädel des Implantatempfängers positionieren zu können, ist vorgesehen, dass das Implantat 1, 10 zunächst mittels des ersten Segments 2 am Zahnwurzelbereich (Limbus alveolaris) 11 derart ausgerichtet wird, dass es vollständig oder zumindest annähernd komplementär am Knochen anliegt. Durch eine solch adäquate Ausrichtung des ersten Segments 2 und falls vorhanden des vierten Segments 5 ist gewährleistet, dass dann das zweite und das dritte Segment 3, 4 und falls vorhanden das fünfte Segment 6 ebenfalls in ihren vorbestimmten Positionen vorliegen, um somit dem Chirurgen eine Matrize zur Reposition beispielsweise einzelner Fragmente vorzugeben. Somit bietet das präformierte Implantat 1, 10 eine Art Vorlage, um die Fragmente in ihrer Normposition (idealerweise ist das Ihre Ausgangslage vor dem Eintreten der Fraktur) zu reponieren und zu fixieren. Die Präformation des erfindungsgemäßen Implantats 1, 10 als dreidimensionale Vorlage bietet dabei insbesondere den Vorteil, dass man bei Mehrfragmentfrakturen die einzelnen Knochenfragmente schneller und einfacher regelrecht reponieren und fixieren kann, so dass die ursprüngliche, durch die geometrische Position des Knochens bestimmte Gesichtsform des Implantatempfängers vollständig oder zumindest annähernd vollständig wiederherstellbar ist.

Beide Ausführungsformen des erfindungsgemäßen Implantats 1, 10 umfassen mehrere als Bohrlöcher mit einem Innengewinde 7 ausgebildete Fixierungselemente. Das Implantat 1, 10 kann mittels Schrauben, die mit passenden Außengewinde zu den Innengewinden der Bohrlöcher ausgeformt sind, am Knochen bzw. den Knochenfragmenten fixiert werden, wodurch es zur Reposition und Fixierung der Fragmente verwendet werden kann. Vorzugsweise nutzt die Verbindung zwischen Platte und Schraube unterschiedliche Durchmesser als die Fixierung zwischen Schraube und Knochen. Vorzugsweise ist das erfindungsgemäße Implantat 1, 10 mit dem ThreadLock TS-System verwendbar oder das erfindungsgemäße Implantat 1, 10 umfasst das ThreadLock TS-System.

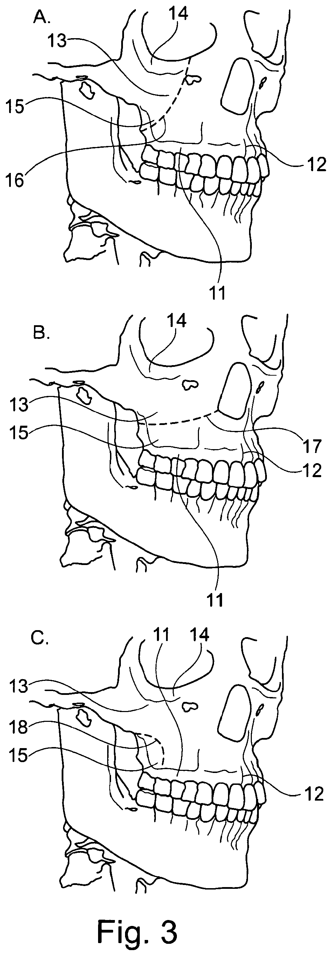

In den Figuren 3A, 3B und 3C sind mehrere potentielle Frakturlinien bei zentrolateralen Mittelgesichtsfrakturen dargestellt. In Figur 3A. ist anhand der gestrichelten Linie der mögliche Verlauf einer pyramidalen Le Fort-II-Fraktur 16 angedeutet - eine zentrale Mittelgesichtsfraktur mit einer pyramidenförmigen Oberkieferabsprengung (Pyramidalfraktur) des Oberkiefermassives. Die Frakturlinie 16 verläuft zumeist entlang oder unterhalb der Sutura frontomaxillaris (mit oder ohne Beteiligung des Nasenskeletts), durch den Processus frontalis maxillae, an der medialen Wand der Orbita (mediale Augenwinkel) entlang, durch das Os lacrimale, durch den Orbitaboden 14, die untere Orbitakante, durch das Foramen infraorbitale oder nahe daran vorbei, schräg nach unten durch die vordere Wand der Kieferhöhle. Die Frakturlinie 16 zieht sich ferner unter dem Jochbein entlang der Fissura pterygomaxillaris und durch die Lamina pterygoidea medialis und lateralis.

In Figur 3B ist anhand der gestrichelten Linie der mögliche Verlauf einer horizontalen Mittelgesichtsfraktur des Typs Le Fort-I dargestellt. Bei der horizontalen Le-Fort-I-Fraktur reicht die Frakturlinie 17 von der Nasenscheidewand zur lateralen Kante der Apertura piriformis, horizontal knapp oberhalb der Wurzelspitzen der Oberkieferzähne. Sie kreuzt zumeist unterhalb der Sutura zygomaticomaxillaris (der Knochennaht zwischen Oberkiefer und Jochbein), quert die Sutura pterygomaxillaris und weiter die Processus pterygoidei lateralis und medialis (jeweils linke und/oder rechte Gesichtshälfte).

In Figur 3C ist anhand der gestrichelten Linie der mögliche Verlauf einer Jochbeinfraktur (lateralen Mittelgesichtsfraktur) 18 dargestellt. Die Frakturlinie 18 verläuft zumeist entlang der Knochennaht (Sutur) zwischen Jochbein und Stirnbein (Sutura zygomaticofrontalis), der seitlichen Wand der knöchernen Augenhöhle 14 (Orbita), entlang einer Knochenspalte im Boden der Orbita (Fissura orbitalis inferior), dem Foramen infraorbitale (Austrittspunkt des Nervus infraorbitalis aus dem Oberkiefer (Maxilla)), der oberen seitlichen und die hinteren Wand der Kieferhöhle (Sinus maxillaris).

Die Verlagerung (Dislokation) von Bruchstücken des Jochbeins kann zur Mitte hin (nach medial) in die Kieferhöhle und Orbita hinein sowie nach unten (kaudal) und hinten (dorsal) erfolgen. Sehr oft liegt bei der operativen Freilegung der Frakturlinien eine zertrümmerte seitliche (laterale) Wand der Kieferhöhle vor.

In den Figuren 3A, 3B und 3C sind ferner die Jochbeinprominenz (Processus zygomaticus) 13, der Stegbereichs zwischen Oberkiefer und Jochbein (Crista zygomaticoalveolaris) 15, der Zahnwurzelbereich (Limbus alveolaris) 11 und die Spina nasalis 12 angedeutet.

## Patentansprüche

1. Implantat (1, 10) zur Reposition und Fixation einer zentrolateralen Mittelgesichtsfraktur wobei das Implantat (1, 10) mehrere präformierte Segmente umfasst, wobei die Form eines ersten Segments (2), welche sich aus dem Abgleich der Schädelgröße und der ethnischen Herkunft eines Implantatempfängers ergibt, derart ausgebildet ist, dass seine am Knochen anzuliegende Unterseite (9) mit der anatomischen Struktur eines Teilbereiches der Außenseite eines Oberkieferknochens komplementär ist, wobei die Form eines zweiten Segments (3), welche sich aus dem Abgleich der Schädelgröße und der ethnischen Herkunft des Implantatempfängers ergibt, derart ausgebildet ist, dass seine am Knochen anzuliegende Unterseite (9) mit der außenseitigen anatomischen Struktur eines Stegbereichs zwischen Oberkiefer und Jochbein komplementär ist, und wobei die Form eines dritten Segments (4), welche sich aus dem Abgleich der Schädelgröße und der ethnischen Herkunft des Implantatempfängers ergibt, derart ausgebildet ist, dass seine am Knochen anzuliegende Unterseite (9) mit der anatomischen Struktur zumindest eines außenseitigen Teilbereiches eines Jochbeins komplementär ist, wobei das Implantat (1, 10) plattenförmig ausgestaltet ist und eine abgerundete Berandung aufweist.

2. Implantat (1, 10) nach Anspruch 1, **dadurch gekennzeichnet, dass** die Form des ersten Segments (2) derart ausgeformt ist, dass seine am Knochen anzuliegende Unterseite (9) mit der anatomischen Struktur des Bereichs, welcher oberhalb des Alveolar Fortsatzes des Oberkiefers angrenzt komplementär ist.

3. Implantat (1, 10) nach Anspruch 1 oder 2, **dadurch gekennzeichnet, dass** die Form des zweiten Segments (3) derart ausgeformt ist, dass seine am Knochen anzuliegende Unterseite (9) mit der anatomischen Struktur des lateralen Jochbeinpfeilers komplementär ist.

4. Implantat (1, 10) nach einem der Ansprüche 1 bis 3, **dadurch gekennzeichnet, dass** die Form des dritten (4) Segments derart ausgeformt ist, dass seine am Knochen anzuliegende Unterseite (9) mit der anatomischen Struktur der Jochbeinprominenz komplementär ist.

5. Implantat (1, 10) nach einem der Ansprüche 1 bis 4, **dadurch gekennzeichnet, dass** das Implantat (1, 10) ein viertes Segment (5) umfasst, wobei die Form des vierten Segments (5), welche sich aus dem Abgleich der Schädelgröße und der ethnischen Herkunft eines Implantatempfängers ergibt, derart ausgebildet ist, dass seine am Knochen anzuliegende Unterseite (9) mit der anatomischen Struktur eines Teilbereiches der Außenseite eines Oberkieferknochens vollständig oder teilweise komplementär ist, und/oder dass das Implantat (1, 10) ein fünftes Segment (6) umfasst, wobei die Form des fünften Segments (6), welche sich aus dem Abgleich der Schädelgröße und der ethnischen Herkunft eines Implantatempfängers ergibt, derart ausgebildet ist, dass seine am Knochen anzuliegende Unterseite (9) mit der außenseitigen anatomischen Struktur eines Teilbereiches eines paranasalen Stegbereichs zwischen Oberkiefer und Jochbein komplementär ist.

6. Implantat (1, 10) nach einem der Ansprüche 1 bis 5, **dadurch gekennzeichnet, dass** zur Determination der jeweiligen Größe der Implantatsegmente die Schädelgröße des Implantatempfängers berücksichtigt wird, wobei drei Größen eines präformierten Implantats pro ethnische Gruppe bereitgestellt sind, sodass anhand dieser Implantate jegliche anatomische Struktur der zentrolateralen Mittelgesichtsknochen wiederherstellbar sind.

7. Implantat (1, 10) nach einem der Ansprüche 1 bis 6, **dadurch gekennzeichnet, dass** das Implantat (1, 10) ein Fixierungselement oder mehrere Fixierungselemente umfasst, wobei das eine Fixierungselement oder die Fixierungselemente einen Fixierungsabschnitt oder mehrere Fixierungsabschnitte, vorzugsweise ein Loch oder mehrere Löcher (7), insbesondere mit einem Innengewinde oder mehreren Innengewinden, und zumindest eine Schraube oder mehrere Schrauben, vorzugsweise ausgestattet mit zumindest einem in das Innengewinde des zumindest einen Loches (7) passenden Außengewindes oder Außengewindeabschnitts, umfasst, und wobei das zumindest eine Fixierungselement oder die Fixierungselemente zur Fixierung und Reposition zumindest eines Knochenfragments, vorzugsweise mehrerer Knochenfragmente, ausgebildet ist oder sind.

8. Implantat (1, 10) nach einem der Ansprüche 1 bis 7, **dadurch gekennzeichnet, dass** das Implantat (1, 10) aus Titan und/oder einer Titanlegierung und/oder einem oder mehreren Kunststoffen, insbesondere einem oder mehreren resorbierfähigen Materialien, hergestellt ist.

9. Implantat (1, 10) nach einem der Ansprüche 1 bis 8, **dadurch gekennzeichnet, dass** die Vorderseite (8) des Implantats (1, 10) derart ausgebildet ist, dass sie an die außenseitigen anatomischen Strukturen des Implantatempfängers angepasst ist und somit die Vorderseite (8) und die Unterseite (9) komplementär ausgebildet sind.

10. Implantat (1, 10) nach einem der Ansprüche 1 bis 9, **dadurch gekennzeichnet, dass** das Implantat (1, 10) eine Dicke von 0,2 mm bis 1,5 mm, vorzugsweise von 0,3 mm bis 0,7 mm aufweist.

## Claims

1. Implant (1, 10) for repositioning and fixation of a centrolateral midfacial fracture, wherein the implant (1, 10) comprises several pre-formed segments, wherein the shape of a first segment (2) resulting from matching of the skull size and the ethnic origin of an implant recipient is designed such that the underside (9) thereof to be adjacent to the bone is complementary with the anatomical structure of one partial region of the outer side of a maxilla bone, wherein the shape of a second segment (3) resulting from matching of the skull size and the ethnic origin of the implant recipient is designed such that the underside (9) thereof to be adjacent to the bone is complementary with the outer anatomical structure of a ridge region between maxilla bone and malar bone, and wherein the shape of a third segment (4) resulting from matching of the skull size and the ethnic origin of the implant recipient is designed such that the underside (9) thereof to be adjacent to the bone is complementary with the anatomical structure of at least one outer partial region of a malar bone, wherein the implant (1, 10) is plate shaped and has a rounded boundary.

2. Implant (1, 10) according to Claim 1, **characterized in that** the shape of the first segment (2) is configured such that the underside (9) thereof to be adjacent to the bone is complementary with the anatomical structure of the region which adjoins above the alveolar extension of the maxilla.

3. Implant (1, 10) according to Claim 1 or 2, **characterized in that** the shape of the second segment (3) is configured such that the underside (9) thereof to be adjacent to the bone is complementary with the anatomical structure of the lateral malar bone column.

4. Implant (1, 10) according to one of Claims 1 to 3, **characterized in that** the shape of the third segment (4) is configured such that the underside (9) thereof to be adjacent to the bone is complementary with the anatomical structure of the zygomatic process of the malar bone.

5. Implant (1, 10) according to one of Claims 1 to 4, **characterized in that** the implant (1, 10) comprises a fourth segment (5), wherein the shape of the fourth segment (5) resulting from matching of the skull size and the ethnic origin of an implant recipient is designed such that the underside (9) thereof to be adjacent to the bone is completely or partially complementary with the anatomical structure of one partial region of the outer side of a maxilla bone, and/or that the implant (1, 10) comprises a fifth segment (6), wherein the shape of the fifth segment (6) resulting from matching of the skull size and the ethnic origin of an implant recipient is designed such that the underside (9) thereof to be adjacent to the bone is complementary with the outer anatomical structure of one partial region of a paranasal ridge region between maxilla bone and malar bone.

6. Implant (1, 10) according to one of Claims 1 to 5, **characterized in that** for determining the respective size of the implant segments, the skull size of the implant recipient is taken into account, wherein three sizes of a pre-formed implant per ethnic group are provided so that using these implants, any anatomical structure of the centrolateral midfacial bones can be restored.

7. Implant (1, 10) according to one of Claims 1 to 6, **characterized in that** the implant (1, 10) comprises a fixation element or several fixation elements, whereby the one fixation element or the fixation elements comprise a fixation section or several fixation sections, preferably a hole or several holes (7), in particular with an internal thread or several internal threads, and at least one screw or several screws, preferably fitted with at least one external thread or external thread section which fits in the internal thread of the at least one hole (7), and wherein the at least one fixation element or the fixation elements is or are designed for the fixation and repositioning of at least one bone fragment, preferably several bone fragments.

8. Implant (1, 10) according to one of Claims 1 to 7, **characterized in that** the implant (1, 10) is made of titanium and/or a titanium alloy and/or one or more plastics, in particular one or more materials with resorption capacity.

9. Implant (1, 10) according to one of Claims 1 to 8, **characterized in that** the front side (8) of the implant (1, 10) is designed such that it is adapted to the outer anatomical structures of the implant recipient and thus the front side (8) and the underside (9) are formed to be complementary.

10. Implant (1, 10) according to one of Claims 1 to 9, **characterized in that** the implant (1, 10) exhibits a thickness of 0.2 mm to 1.5 mm, preferably 0.3 mm to 0.7 mm.

## Revendications

1. Implant (1, 10) destiné au repositionnement ou à la fixation d'une fracture du visage médian centrolatéral dans lequel l'implant (1, 10) comprend plusieurs segments préformés, dans lequel la forme d'un premier segment (2), laquelle résulte de la comparaison de la taille du crâne et de l'origine ethnique d'un receveur de l'implant, est conçue de telle manière que sa face inférieure (9) adjacente à l'os est complémentaire à la structure anatomique d'une partie de la face extérieure d'un os de mâchoire supérieure, dans lequel la forme d'un deuxième segment (3), laquelle résulte de la comparaison de la taille du crâne et de l'origine ethnique du receveur de l'implant, est conçue de telle manière que sa face inférieure (9) adjacente à l'os est complémentaire à la structure anatomique extérieure d'une zone de liaison entre la mâchoire supérieure et l'os malaire, et dans lequel la forme d'un troisième segment (4), laquelle résulte de la comparaison de la taille du crâne et de l'origine ethnique du receveur de l'implant, est conçue de telle manière que sa face inférieure (9) adjacente à l'os est complémentaire à la structure anatomique d'au moins une partie extérieure d'un os malaire, dans lequel l'implant (1, 10) est agencé en forme de plaque et présente un bord arrondi.

2. Implant (1, 10) selon la revendication 1, **caractérisé en ce que** la forme du premier segment (2) est réalisée de telle manière que sa face inférieure (9) adjacente à l'os est complémentaire à la structure anatomique de la zone, laquelle est contiguë au-dessus du prolongement alvéolaire de la mâchoire supérieure.

3. Implant (1, 10) selon la revendication 1 ou 2, **caractérisée en ce que** la forme du deuxième segment (3) est réalisé de telle manière que sa face inférieure (9) adjacente à l'os est complémentaire à la structure anatomique du pilier latéral de l'os malaire.

4. Implant (1, 10) selon l'une quelconque des revendications 1 à 3, **caractérisé en ce que** la forme du troisième segment (4) est réalisée de telle manière que sa face inférieure (9) adjacente à l'os est complémentaire à la structure anatomique de la proéminence de l'os malaire.

5. Implant (1, 10) selon l'une quelconque des revendications 1 à 4, **caractérisé en ce que** l'implant (1, 10) comprend un quatrième segment (5), dans lequel la forme du quatrième segment (5), laquelle résulte de la comparaison de la taille du crâne et de l'origine ethnique d'un receveur de l'implant, est conçue de telle manière que sa face inférieure (9) adjacente à l'os est complémentaire totalement ou partiellement à la structure anatomique d'une partie de la face extérieure d'un os de mâchoire supérieure, et/ou que l'implant (1, 10) comprend un cinquième segment (6), dans lequel la forme du cinquième segment (6), laquelle résulte de la comparaison de la taille du crâne et de l'origine ethnique d'un receveur de l'implant, est conçue de telle manière que sa face inférieure (9) adjacente à l'os est complémentaire à la structure anatomique extérieure d'une partie d'une zone de liaison paranasale entre la mâchoire supérieure et l'os malaire.

6. Implant (1, 10) selon l'une quelconque des revendications 1 à 5, **caractérisé en ce que** pour la détermination de la taille respective des segments de l'implant la taille du crâne du receveur de l'implant est prise en compte, dans lequel trois tailles d'un implant préformé sont prévues par groupe ethnique, de sorte que à l'aide de ces implants toute structure anatomique de l'os du visage médian centrolatéral peuvent être reproduites.

7. Implant (1, 10) selon l'une quelconque des revendications 1 à 6, **caractérisé en ce que** l'implant (1, 10) comprend un élément de fixation ou plusieurs éléments de fixation, dans lequel ledit un élément de fixation ou lesdits éléments de fixation comprennent une section de fixation ou plusieurs sections de fixation, de préférence un trou ou plusieurs trous (7), en particulier avec un filetage intérieur ou plusieurs filetages intérieurs, et au moins une vis ou plusieurs vis, de préférence dotés d'au moins un filetage extérieur ou des sections de filetage extérieur ajusté(es) dans le filetage intérieur de l'au moins un trou (7), et dans lequel l'au moins un élément de fixation ou les éléments de fixation est(sont) conçu(s) pour la fixation ou le repositionnement d'au moins un fragment osseux, de préférence plusieurs fragments osseux.

8. Implant (1, 10) selon l'une quelconque des revendications 1 à 7, **caractérisé en ce que** l'implant (1, 10) est fabriqué à partir de titane et/ou d'un alliage de titane et/ou d'une ou plusieurs matières plastiques, en particulier d'un ou plusieurs matériaux résorbables.

9. Implant (1, 10) selon l'une quelconque des revendications 1 à 8, **caractérisé en ce que** la face avant (8) de l'implant (1, 10) est conçue de telle manière qu'elle est ajustée à la structure anatomique extérieure du receveur de l'implant et que la face avant (8) et la face inférieure (9) sont donc conçues de manière complémentaire.

10. Implant (1, 10) selon l'une quelconque des revendications 1 à 9, **caractérisé en ce que** l'implant (1, 10) présente une épaisseur allant de 0,2 mm à 1,5 mm, de préférence de 0,3 mm à 0,7 mm.
